# EUROPEAN PATENT APPLICATION

(11) **EP 3 545 918 A1**
(43) Date of publication of application: **02.10.2019**
(21) Application number: 17874526.1
(22) Date of filing: 07.10.2017
(51) Int. Cl.: A61F 5/37

(54) **MALE URINARY INCONTINENCE PREVENTION TOOL**

(30) Priority: 22.11.2016 JP 2016226904
(71) Applicant: Nishiyama, Tetsuryuu, Hakusan-shi, Ishikawa 920-2166 (JP)
(72) Inventor: Nishiyama, Tetsuryuu, Hakusan-shi, Ishikawa 920-2166 (JP)
(74) Representative: FRKelly
(86) International application number: PCT/JP2017/036556
(87) International publication number: WO 2018/096809

(57) **Abstract**

There is provided an instrument for preventing male urinary incontinence that is safe and sanitary, and can be equipped easily and correctly, while the skin is prevented from being lodged in the instrument.

The urinary incontinence prevention instrument 1 for males of the present invention includes: an annular part 10 made by annularly forming a portion of a belt-like component 11; a convex part 20 for pressing the urethra 101, convex parts attached to the inner circumference surface side of the annular part; and an adjusting mechanism 40 for adjusting the diameter of the annular part. The instrument includes a lodge-proof prevention component 50 having a hole 51 for passing the belt-like component. The lodge-proof prevention component, positioned between the penis and the adjustment mechanism, prevents the skin of the penis 100 from being lodged in the adjustment mechanism.

## Description

### TECHNICAL FIELD

The present invention relates to an instrument for preventing male urinary incontinence that is safe and sanitary, and one that can be equipped easily and correctly, while preventing the skin from becoming lodged in the instrument.

### BACKGROUND

The causes of male urinary incontinence are various. For example, it can be aging, prostatic hypertrophy, prostatic cancer, diabetes, being overweight, or neurological symptoms such as multiple sclerosis, Parkinson's disease, and Alzheimer's disease. These are the problems which many people suffer from.

Conventionally, instruments shown in Patent Documents 1 through 3 are known as instruments for preventing male urinary incontinence.

Each of these instruments is formed like a ring having a partial opening, and has a convex part in the lower area on the inner circumference surface. The urethra is located in the center lower part of the penis, and stretches from the root to the tip. The user of the instrument expands the opening of the instrument for inserting the penis inside, and then tightens the opening in a state where the convex part is contacted to the urethra. Since the urethra is pushed by the convex part and is blocked, urinary incontinence can be prevented.

### CITATION LIST

### PATENT LITERATURE

Patent Document 1: JP 2002 - 512841 A1
Patent Document 2: JP 2001 - 501116 A1
Patent Document 3: JP Sho58 - 175554 A1

### SUMMARY OF INVENTION

### TECHNICAL PROBLEM

However, the technology disclosed in Patent Documents 1 through 3 has the following problems.

That is, when the user tightens the opening, one end of the ring moves along the inner surface of the other end. In this case, the skin of the penis can be lodged in a space between one end and the other end, and there is a problem that the user may feel pain from or be injured by. Such a problem can occur if the size of the penis changes when the instrument is equipped onto the penis, or when the penis moves in the back-and-forth direction.

In Patent Document 1, this problem is resolved by fitting a padded material made of sponge to the inner circumference surface (see [0063] and Figs. 12 and 13 of Patent Document 1). However, there is a sanitary problem that makes urine seep into the padded material, or the skin steamed due to the decrease of air permeability.

When the user starts to close the opening in a state where the convex part is contacted to the lower part of the urethra, there is a problem where the position of the convex part is gradually displaced from the urethra, in the case that one end of the ring moves along the inner surface of the other end. This problem is significant when the user attaches the instrument onto the penis using only one hand, as in Patent Document 1 (see [0016] of Patent Document 1).

In the attached state, the entire inner circumference surface of the instrument adheres to the penis. Thus, there is a problem where a sufficient amount of blood flow cannot be maintained because the blood vessels in the penis are pressed.

The object of the present invention is to provide an instrument for preventing male urinary incontinence that is safe and sanitary and can be worn easily and correctly in view of the previously mentioned circumstances, while the skin is prevented from being lodged in the instrument.

### SOLUTION TO PROBLEM

The urinary incontinence prevention instrument for males according to the present invention includes: an annular part made by annularly forming a portion of a belt-like component; a convex part for pressing the urethra, the convex part being attached to the inner circumference surface side of the annular part; and an adjusting mechanism for adjusting the diameter of the annular part. The instrument comprises a lodge-proof component having a hole for passing the belt-like component. This component, positioned between the penis and the adjustment mechanism, prevents the penis from being lodged in the adjustment mechanism.

The instrument can be provided with a plurality of convex parts for the spacer. The space is formed between the penis and the inner circumference surface, when the convex parts are attached to the inner circumference surface of the annular part.

The adjustment mechanism can be configured to change the length of both ends of the belt-like component in an equivalent amount.

The circumference of the annular part can be covered with a double cloth.

### ADVANTAGEOUS EFFECTS OF INVENTION

In the present invention, the lodge-proof component prevents the skin of the penis from being lodged in the adjustment mechanism, and safety can thereby be maintained.

The convex parts for the spacer allows for forming a space between the penis and the inner circumference surface of the annular part. Thus, the blood-flow and the air permeability of the penis are maintained, and safety and sanitation are secured.

The use of the adjustment mechanism that can change the length of both ends of the belt-like component in an equivalent amount prevents the position of the convex part for pressing the urethra from moving from the lowermost annular part, when the diameter of the annular part is reduced. In this way, the instrument can be equipped easily and correctly with one hand, in a state where the convex part for pressing the urethra is in contact with the urethra.

Since the circumference of the annular part is covered with double cloth, the cloth can be moved together with the skin of the penis, when the penis moves inside the annular part in the back-and-forth direction. Thus, the skin can be prevented from being rubbed onto the inner surface of the annular part and the user is free from pain or discomfort.

### BRIEF DESCRIPTION OF DRAWINGS

Fig. 1 is a perspective diagram illustrating the appearance of the urinary incontinence prevention instrument, before the instrument is tightened by the adjustment mechanism.
Fig. 2 is a perspective diagram illustrating the state of the urinary incontinence prevention instrument, when the instrument is tightened by the adjustment mechanism.
Fig. 3 is the front view illustrating the usage of the urinary incontinence prevention instrument. Fig. 3A shows the state before the instrument is tightened by the adjustment mechanism. Fig. 3B shows the state when the instrument is tightened.
FIG.4 is a perspective diagram illustrating the state where the circumference of the annular part is covered with double cloth.

### DESCRIPTION OF EMBODIMENTS

The embodiments of the urinary incontinence prevention instrument according to the present invention will be explained.

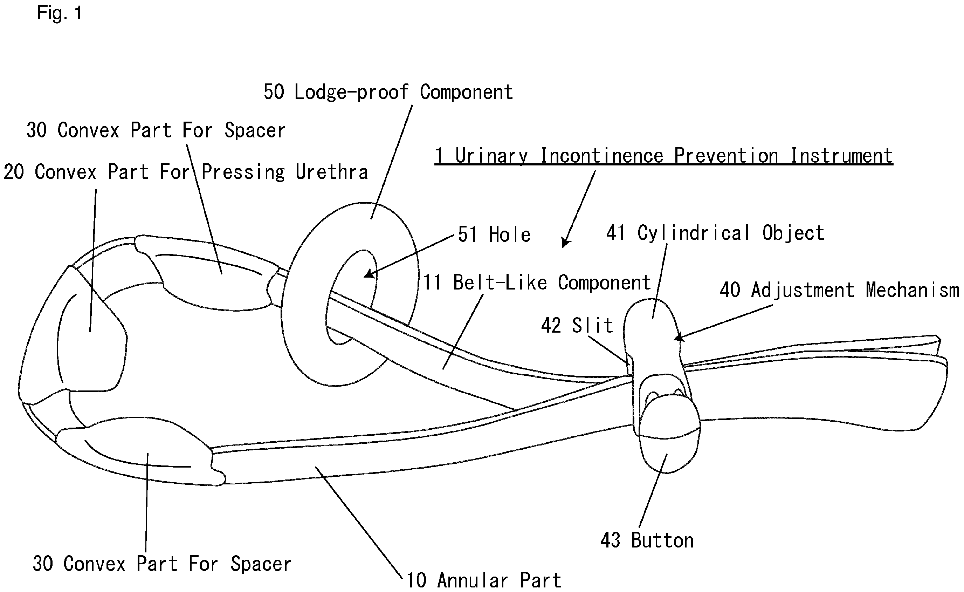

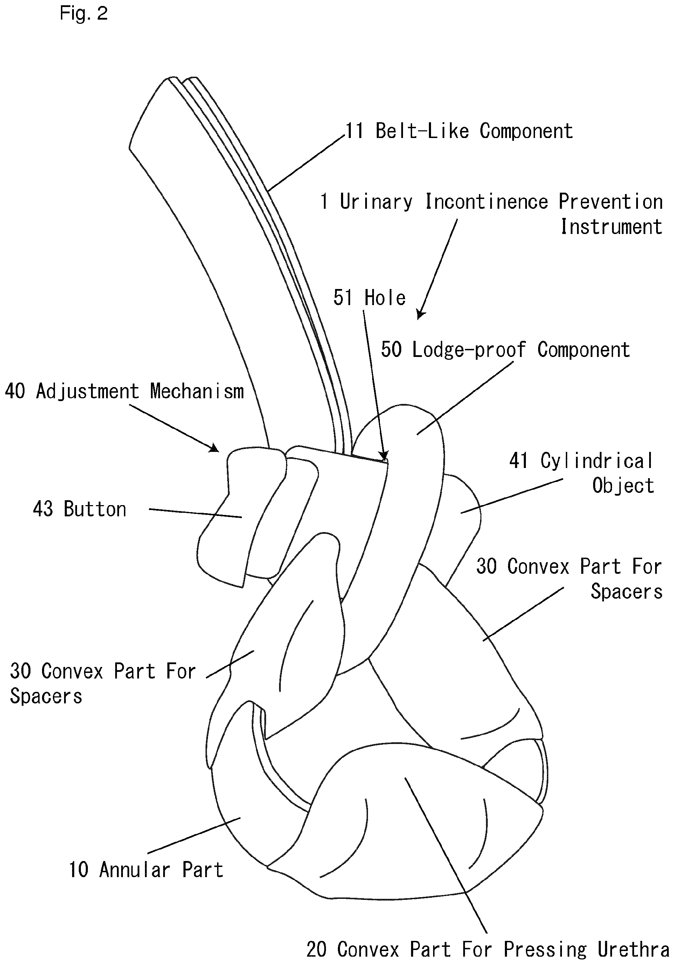

As illustrated in Fig. 1 and Fig. 2, the urinary incontinence prevention instrument 1 comprises an annular part 10, a convex part 20 for pressing the urethra, a convex part 30 for the spacer, an adjustment mechanism 40, and a lodge-proof component 50.

The annular part 10 is constructed by forming annularly a portion of a belt-like component 11. The material of the belt-like component 11 is not specifically limited, but can be made of textiles, nonwoven fabric, synthetic resin, or paper, for example.

The convex part 20 for pressing the urethra is a component for pressing the urethra 101. Since the urethra 101 is positioned in the center lower part of the penis 100, the convex part 20 is fitted to the lower part of the inner circumference surface of the annular part 10. The convex part 20 is preferably formed with a material which is elastically deformable and water resistant, such as a sponge.

The convex part 30 for the spacer is a component for forming a space between the penis 100 and the inner circumference surface of the annular part 10. In the embodiment of the present invention, two convex parts 30 are fitted to the inner circumference surface of the annular part 10; however, three or more convex parts 30 can be equipped.

The shape of the convex part 20 for pressing the urethra and the convex part 30 for the spacer is not specifically limited. However, a semi-circular shape is preferable because the convex part can be contacted to the penis 100 on a spherical surface. This reduces stimulus to the penis 100 and allows wearing it for many hours.

The convex part 20 for pressing the urethra and the convex parts 30 for the spacer may be provided integrally with the belt-like component 11 using a frame.

The adjustment mechanism 40 is a component for adjusting the diameter of the annular part 10. The adjustment mechanism 40 comprises: a cylindrical object 41; a slit 42 formed in a portion of the cylindrical object 41; a stopper accommodated inside the cylindrical object 41 (not illustrated), the stopper being energized with a spring such that the stopper is movable to the direction for closing the slit 42; and a button 43 for moving the stopper for resisting the spring force.

When the user pushes the button 43, the slit 42 opens and the belt-like component 11 becomes movable inside the slit 42. When the button 43 is released, the stopper moves by the spring force to the direction for closing the slit 42, and the stopper presses the belt-like component 11 so that this component is unmovable. The diameter of the annular part 10 can be adjusted by adjusting the position of the adjustment mechanism 40 in a state where the user pushes the button 43.

According to this adjustment mechanism 40, the position of the convex part 20 for pressing the urethra does not move from the lowermost annular part 10 during the adjustment of the diameter of the annular part 10 because the length between both ends of the belt-like component 11 changes in an equivalent amount. Thus, the convex part 20 for pressing the urethra can be correctly contacted to the urethra 101.

The lodge-proof component 50 is a component for preventing the skin of the penis 100 from being lodged in the adjustment mechanism 40, since the lodge-proof component 50 is positioned between the penis 100 and the adjustment mechanism 40.

The lodge-proof component 50 of the present embodiment is formed with a circular component made of rubber having a hole 51 for passing the belt-like component 11. The lodge-proof component 50 does not have to be circular shaped, and can be polygonal. The lodge-proof component 50 can be formed with a flat plate having a hole 51 therein. The material of the stuck-state prevention component 50 is not specifically limited to rubber, but can be wood, metal, or synthetic resins such as silicone.

Next, the usage of the urinary incontinence prevention instrument 1 will be explained.

The user opens the slit 42 by pushing the button 43 of the adjustment mechanism 40, and expands the diameter of the annular part 10 before use, by moving the adjustment mechanism 40 to the end side of the belt-like component 11.

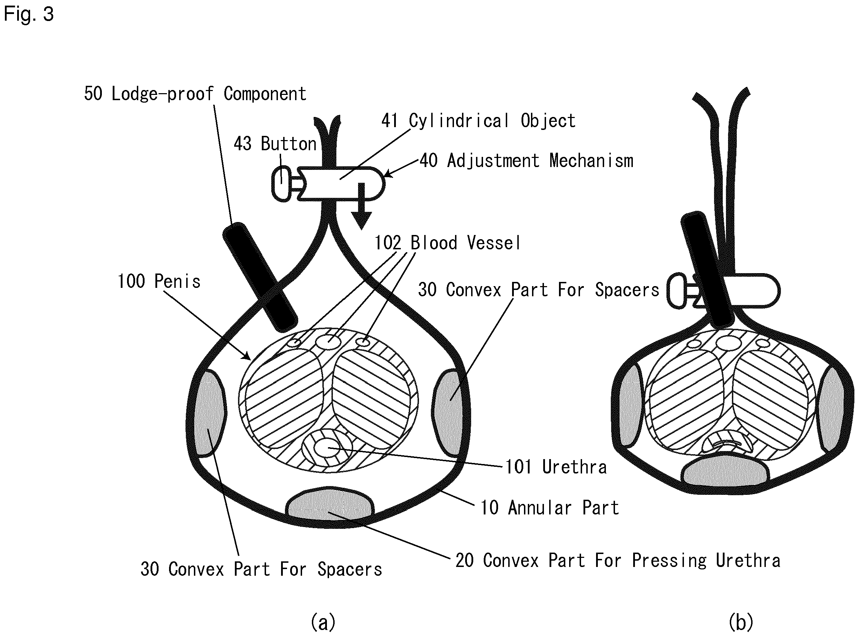

Then, as illustrated in Fig. 3A, the penis 100 is inserted into the annular part 10 in a state where the convex part 20 for pressing the urethra is positioned at the lowermost part. At this point, the lodge-proof component 50 is moved to the upper part of the penis 100 (near the adjustment mechanism 40). In Fig. 3, the sectional view of the penis 100 is shown.

Then, the user pushes the button 43, and moves the adjustment mechanism 40 toward the annular part 10 (to the direction of the arrow in the figure) for reducing the diameter of the annular part 10, and tightens it until the convex part 20 for pressing the urethra moderately presses the urethra 101. The adjustment mechanism 40 can be moved with only one hand, if the user pushes the button 43 with the right thumb, supports the bottom of the cylindrical object 41 with the index finger, and pulls the end part of the belt-like component 11 with the, ring finger and little finger.

When the diameter of the annular part 10 is reduced, the lodge-proof component 50 stays in the position between the penis 100 and the adjustment mechanism 40. Thus, the skin of the penis 100 is prevented from being lodged in the adjustment mechanism 40.

As illustrated in Fig. 3B, in a state where wearing the urinary incontinence prevention instrument 1 on the penis 100 is completed, the urethra 101 is moderately pressed by the convex part 20 for pressing the urethra, and a moderate space is provided between the penis 100 and the inner circumference surface of the annular part 10 by the convex part 30 for the spacer. Thus, the air permeability of the skin of the penis 100 and the blood-flow of the blood vessel 102 are maintained, while urinary incontinence is prevented.

When the urinary incontinence prevention instrument 1 is worn on the penis 100, the lodge-proof component 50 stays in position between the penis 100 and the adjustment mechanism 40. Thus, the skin of the penis 100 is prevented from being lodged in the adjustment mechanism 40.

By attaching the lodge-proof component 50 to the adjustment mechanism 40 using the hole 51, unintentional movement of the adjustment mechanism 40 toward the end part of the belt-like component 11 is avoided with the lapse of time, and it can be prevented that the diameter of the annular part 10 becomes large and the part loosens.

The user can adjust the strength of tightening according to various situations. For example, the user can fix it tighter when performing heavy labor, and looser when sitting in a chair.

In the present embodiment, the urinary incontinence prevention instrument 1 has been assumed to have the convex part 30 for the spacer. However, instead of equipping the convex part 30 for the spacer in the urinary incontinence prevention instrument 1, blood-flow of the blood vessel 102 can be maintained by configuring the belt-like component 11 with a material having high air permeability or high flexibility.

In the previous condition, the adjustment mechanism 40 is configured to change both ends of the belt-like component 11 in an equivalent amount, but not be limited to this. For example, like the buckle of a belt, the diameter of the annular part 10 can be changed by pulling only one end of the belt-like component 11. In this case, the convex part 20 for pressing the urethra needs to be correctly in contact with the urethra 101.

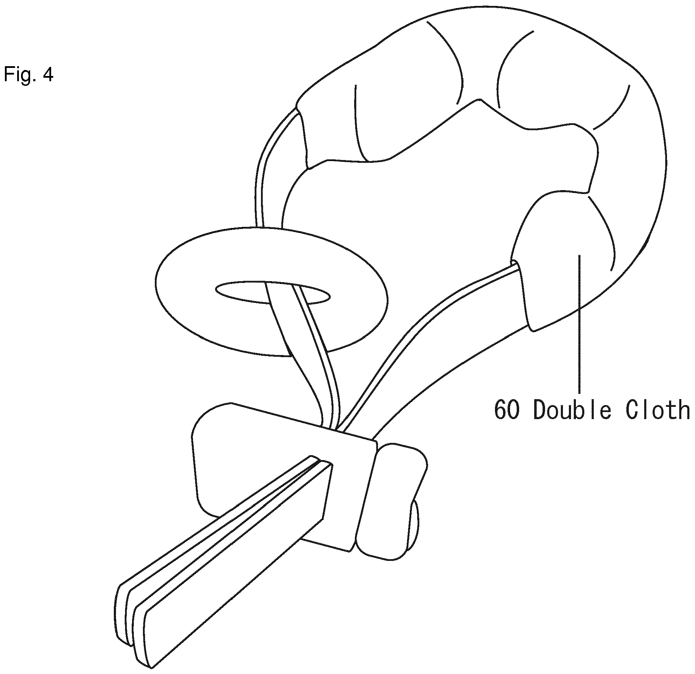

The circumference of the annular part 10 can be covered with a double cloth 60 made of front side cloth and back side cloth, as shown in Fig. 4. When the penis 100 moves in the back-and-forth direction within the annular part 10, the front side cloth moves together with the skin of the penis 100, and the back side cloth stays on the surfaces of the convex part 20 for pressing the urethra and the convex part 30 for the spacer if the annular part 10 is covered with the double cloth 60. Thus, the skin can be prevented from being rubbed and the user can prevent himself from becoming pained or in discomfort. The material of the cloth is not specifically limited; however, the use of silk is preferable in consideration of the feeling of touch.

### INDUSTRIAL APPLICABILITY

The present invention relates to an instrument for preventing male urinary incontinence that is safe and sanitary, and the instrument can be equipped easily and correctly, while the skin is prevented from being lodged in the instrument. The invention is therefore applicable to the industry.

### REFERENCE SIGN LIST

- 1: urinary incontinence prevention instrument
- 10: annular part
- 11: belt-like component
- 20: convex part for pressing urethra
- 30: convex part for spacer
- 40: adjustment mechanism
- 41: cylindrical object
- 42: slit
- 43: button
- 50: lodge-proof component.
- 51: hole
- 60: double cloth
- 100: penis
- 101: urethra
- 102: blood vessel

## Claims

1. An urinary incontinence prevention instrument for males including: an annular part made by annularly forming a portion of a belt-like component; a convex part for pressing the urethra, the convex part being attached to the inner circumference surface side of the annular part; and an adjusting mechanism for adjusting the diameter of the annular part, the urinary incontinence prevention instrument comprising:
a lodge-proof component having a hole for passing the belt-like component, wherein
the lodge-proof component, positioned between the penis and the adjustment mechanism, prevents the penis from being lodged in the adjustment mechanism.

2. The urinary incontinence prevention instrument as claimed in claim 1, the instrument comprising:
a plurality of convex parts for the spacer, wherein a space is formed between the penis and the inner circumference surface when the convex parts are attached to the inner circumference surface of the annular part.

3. The urinary incontinence prevention instrument as claimed in claim 1 or 2, wherein,
the adjustment mechanism is configured to change the length of both ends of the belt-like component in an equivalent amount.

4. The urinary incontinence prevention instrument as claimed in one of claims 1 through 3, wherein,
the circumference of the annular part is covered with a double cloth.
